# EUROPÄISCHE PATENTSCHRIFT

(11) **EP 3 769 661 B1**
(45) Veröffentlichungstag und Bekanntmachung des Hinweises auf die Patenterteilung: **07.05.2025**
(21) Anmeldenummer: 20185458.5
(22) Anmeldetag: 13.07.2020
(51) Int. Cl.: A61B 1/00, A61B 1/07, G16H 40/40, G01M 11/00

(54) **MEDIZINISCHES SYSTEM UND VERFAHREN ZUM ERMITTELN EINES WARTUNGSBEDARFS EINER LICHTLEITKOMPONENTE DES MEDIZINISCHEN SYSTEMS**
MEDICAL SYSTEM AND METHOD FOR DETERMINING A MAINTENANCE REQUIREMENT OF AN OPTICAL WAVEGUIDE COMPONENT OF THE MEDICAL SYSTEM
SYSTÈME MÉDICAL ET PROCÉDÉ DE DÉTERMINATION D'UN BESOIN DE MAINTENANCE D'UN COMPOSANT DE CONDUCTEUR DE LUMIÈRE DU SYSTÈME MÉDICAL

(30) Priorität: 26.07.2019 DE 102019120281
(43) Veröffentlichungstag der Anmeldung: 27.01.2021
(73) Patentinhaber: OLYMPUS Winter & Ibe GmbH, 22045 Hamburg (DE)
(72) Erfinder: ENDERS, Borg, 21149 Hamburg (DE); GEEST, Arne, 22089 Hamburg (DE); TINGELHOFF, Kathrin, 22303 Hamburg (DE); KÖNIG, Florian, 22926 Ahrensburg (DE)
(74) Vertreter: Seemann & Partner Patentanwälte mbB

(56) Entgegenhaltungen:
- DE-T2- 60 306 309
- US-A1- 2011 193 948
- US-A1- 2016 015 247

## Beschreibung

Die Erfindung betrifft ein medizinisches System umfassend eine Datenverarbeitungseinheit und Lichtleitkomponenten, nämlich eine Lichtquelle, ein Lichtleitkabel, einen Kamerakopf und ein Endoskop, durch die jeweils ein Bündel von Lichtleitfasern geführt ist. Ferner betrifft die Erfindung ein Verfahren zum Ermitteln eines Wartungsbedarfs einer Lichtleitkomponente eines medizinischen Systems, umfassend eine Lichtquelle, ein Lichtleitkabel, einen Kamerakopf und ein Endoskop als Lichtleitkomponenten, wobei durch die Lichtleitkomponenten jeweils ein Bündel von Lichtleitfasern geführt ist.

Medizinische Endoskope werden regelmäßig gewartet, um deren optimale Funktionalität sicherzustellen. Die regelmäßige Wartung stellt sicher, dass die bei häufiger Nutzung und der sich anschließenden Aufbereitung der Endoskope unvermeidbar auftretenden Verschleißerscheinungen sich nicht negativ auf die Funktionalität des Endoskops auswirken. Mögliche Verschleißerscheinungen sind beispielsweise eine verminderte Bildqualität oder auch eine unzureichende Ausleuchtung des Blickfeldes des Endoskops.

Um festzustellen, wann an einem Endoskop die erforderlichen Wartungsarbeiten durchzuführen sind, wird vielfach die Anzahl der Einsätze des Endoskops aufsummiert. Alternativ wird gezählt, wie häufig das Endoskop aufbereitet wurde. Ist ein vorgegebener Maximalwert erreicht, wird das Endoskop zwecks Wartung beispielsweise zum Hersteller geschickt.

Aus der US 4,996,975 A ist ein Endoskop bekannt, bei dem durch Aufsummieren verschiedener Nutzungsdaten ermittelt wird, ob für das Endoskop ein Wartungsbedarf besteht. Zu diesen Nutzungsdaten zählt beispielsweise eine Anzahl von Betätigungen eines bestimmten Schalters. Anhand der Anzahl der Betätigungen dieses Schalters bestimmt ein Algorithmus, ob das Endoskop gewartet werden muss.

Der konkrete Wartungsbedarf des Endoskops lässt sich durch Aufsummieren der Einsätze und Aufbereitungszyklen jedoch nur abschätzen. So ist es möglich, dass ein Endoskop in die Wartung gegeben wird, obwohl es trotz häufiger Nutzung nach wie vor optimal funktioniert. Anderseits kann es vorkommen, dass Endoskope für medizinische Eingriffe eingeplant werden, die keine optimale Funktionalität mehr erreichen.

US 2016/0015247 A1 zeigt ein Verfahren und eine Vorrichtung zur Untersuchung der Licht- und/oder Bildübertragungseigenschaften eines Endoskop- oder Exoskopsystems. Dabei ist es vorgesehen, die vom Endoskop- oder Exoskopsystem ausgestrahlte Lichtstärke zu messen, um die Qualität einer verwendeten Kombination von Komponenten zu bestimmen.

US 2011/0193948 A1 offenbart ein System, mit dem eine drahtlose Verbindung zwischen einem Endoskop und einem Kamerakopf hergestellt wird. Videosignalverarbeitungsparameter des Kamerakopfs werden automatisch eingestellt basierend auf Kenndaten eines verbundenen Endoskops.

Es ist eine Aufgabe der Erfindung, ein medizinisches System und ein Verfahren anzugeben, mit dem ein Wartungsbedarf der Lichtleitkomponenten des medizinischen Systems, insbesondere einer Lichtquelle, eines Lichtleitkabels, eines Kamerakopfes und eines Endoskops des medizinischen Systems zuverlässiger und präziser festgestellt werden kann.

Gelöst wird diese Aufgabe durch ein medizinisches System nach Anspruch 1, umfas-send eine Datenverarbeitungseinheit und Lichtleitkomponenten, nämlich eine Lichtquelle, ein Lichtleitkabel, einen Kamerakopf und ein Endoskop, durch die jeweils ein Bündel von Lichtleitfasern geführt ist, wobei das medizinische System dadurch fortgebildet ist, dass die Datenverarbeitungseinheit dazu eingerichtet ist, einen Wartungsbedarf der Lichtleitkomponenten zu ermitteln, indem sie während eines medizinischen Eingriffs die folgenden Daten erfasst und als historische Daten abspeichert:
- einen Lichtstärkeparameterwert der Lichtquelle, der mit einer während des medizinischen Eingriffs von der Lichtquelle abgegebenen Lichtstärke korreliert,
- einen Lichtleitkomponenten-Parametersatz, der eine Konfiguration der während des medizinischen Eingriffs zum Einsatz kommenden Lichtleitkomponenten beschreibt,
- ein externer Eingriffsparameter, der Art des medizinischen Eingriffs beschreibt,
wobei die Datenverarbeitungseinheit ferner dazu eingerichtet ist, aus den historischen Daten von wenigstens zwei medizinischen Eingriffen mit gleichem Lichtleitkomponenten-Parametersatz und gleichen externen Eingriffsparametern einen Trend des Lichtstärkeparameters zu ermitteln und eine Wartungsbedarfsmeldung zu erzeugen, wenn in dem Trend eine einen vorgebbaren Grenzwert überschreitende Abweichung feststellbar ist, und die Wartungsbedarfsmeldung auszugeben.

Die Erfindung beruht auf der Überlegung, dass ein Wartungsbedarf der Lichtleitkomponenten eines medizinischen Systems, insbesondere des Lichtleitkabels, des Kamerakopfes und/oder des Endoskops vielfach auf eine Beschädigung des in diesen Komponenten vorhandenen Bündels von Lichtleitfasern zurückzuführen ist. Gebrochene Lichtleitfasern führen zu Lichtstreuung innerhalb des Faserbündels und verringern so die Helligkeit, mit der das Blickfeld des Endoskops ausgeleuchtet wird. Um trotz gebrochener Lichtleitfasern eine ausreichende Helligkeit zu erreichen, wird ein Nutzer des Endoskops während eines medizinischen Eingriffs die Lichtstärke der Lichtquelle entsprechend erhöhen. Alternativ wird die Lichtquelle derart betrieben, dass die ausreichendende, vorzugsweise vorbestimmte oder vordefinierte Helligkeit automatisch erreicht wird. Der Wert des Lichtstärkeparameters, der mit der Lichtstärke der Lichtquelle korreliert ist, ist somit ein indirekter Indikator für den Wartungsbedarf der Lichtleitkomponenten. Der Lichtstärkeparameter ist beispielsweise eine Stromstärke eines Stroms, mit der die Lichtquelle betrieben wird.

Um den Wartungsbedarf einer Lichtleitkomponente zu ermitteln, muss diese zunächst identifiziert werden. Zu diesem Zweck wird der Parametersatz, der die Konfiguration der Lichtleitkomponenten beschreibt, erfasst. Dieser Parametersatz beschreibt welche der Lichtleitkomponenten gemeinsam als medizinisches System verwendet werden. Eine Identifizierung der einzelnen Komponenten erfolgt beispielsweise anhand von deren Seriennummer.

Um den Wartungsbedarf zuverlässig ermitteln zu können, muss zudem berücksichtigt werden, dass die Lichtstärke der Lichtquelle nicht nur von der Anzahl der gebrochenen Lichtleitfasern, sondern auch von weiteren, externen Parametern des medizinischen Eingriffs, den externen Eingriffsparametern, abhängt. Der Einfluss der externen Eingriffsparameter auf den Lichtstärkeparameter unterscheidet sich nach Art und/oder der Durchführung des medizinischen Eingriffs. Beispielsweise erfordern manche Arten medizinischer Eingriffe eine stärkere Beleuchtung, so dass bei diesen generell ein höherer Wert für den Lichtstärkeparameter festzustellen ist. Auch in einzelnen Phasen eines medizinischen Eingriffs kann eine stärkere Beleuchtung erforderlich sein. Beide Umstände stehen nicht in Korrelation zu einem Wartungsbedarf der Lichtleiter, so dass sie bei der Datenerfassung und -verarbeitung isoliert werden müssen.

Aus diesem Grund werden als historische Daten jeweils Datensätze gespeichert, die neben dem Lichtstärkeparameterwert auch den zugehörigen Lichtleitkomponenten-Parametersatz und externen Eingriffsparameter umfassen. Aus den historischen Daten von mehreren medizinischen Eingriffen wird anschließend ein Trend des Lichtstärkeparameters berechnet. Bei diesem handelt es sich um einen bereinigten Trend der Lichtstärke der Lichtquelle über mehrere vergleichbare medizinische Eingriffe hinweg, wobei jeweils die Konfiguration der Lichtleitkomponenten berücksichtigt wird.

Zur Berechnung des Trends des Lichtstärkeparameters werden also nur solche Werte des Lichtstärkeparameters berücksichtigt, die unter im wesentlichen identischen Bedingungen erfasst wurden. Auf diese Weise wird verhindert, dass in dem insbesondere auch zeitlichen Verlauf eine durch eine Änderung des wenigstens einen externen Eingriffsparameters oder des Lichtleitkomponenten-Parametersatzes verursachte Änderung des Lichtstärkeparameters auftritt. Wird in dem Trend eine Änderung des Lichtstärkeparameters festgestellt, ist diese Änderung somit auf eine Beschädigung der Lichtleitfasern zurückzuführen. Auf diese Weise wird ein Wartungsbedarf der Lichtleitkomponenten zuverlässig erkannt und eine Wartungsbedarfsmeldung erzeugt.

Die Wartungsbedarfsmeldung wird insbesondere an technisches Personal ausgegeben, das für die Wartung von medizinischen Geräten zuständig ist. Dabei kann es sich um internes oder externes Personal handeln. Die Ausgabe der Wartungsbedarfsmeldung erfolgt insbesondere elektronisch. In diesem Zusammenhang kann eine Nachricht über eine Schnittstelle des medizinischen Systems ausgegeben werden oder es erfolgt im einfachsten Fall die Ansteuerung einer Warnlampe.

Das Endoskop ist insbesondere ein Videoendoskop. Im Kontext der vorliegenden Beschreibung ist unter einem Endoskop sowohl ein Endoskop mit starren Schaft als auch ein Endoskop mit flexiblem Schaft zu verstehen. Die Datenverarbeitungseinheit ist insbesondere ein Computer, Server oder dergleichen.

Gemäß einer Ausführungsform ist das medizinische System dadurch fortgebildet, dass der externe Eingriffsparameter einen oder mehrere der folgenden Parameter umfasst: einen Phasenparameter, der eine Phase des medizinischen Eingriffs kennzeichnet, einen Nutzerparameter, der Nutzerdaten des den medizinischen Eingriff durchführenden medizinischen Personals kennzeichnet, einen Patientenparameter, der für einen Patienten charakteristische Daten umfasst, einen Unterstützungssystemparameter, der wenigstens einen Parameter eines Systems zur medizinischen Entscheidungsunterstützung umfasst.

Es wurde erkannt, dass der Wert des Lichtstärkeparameters während eines medizinischen Eingriffs von bestimmten externen Eingriffsparametern abhängt. Zu diesen externen Eingriffsparametern zählt der Eingriffstypparameter, welcher die Art des medizinischen Eingriffs kennzeichnet. Diese Information ist wichtig, da bei bestimmten Arten von medizinischen Eingriffen eine größere Helligkeit notwendig ist als bei anderen. Auch die Phase des medizinischen Eingriffs spielt eine Rolle für den Wert des Lichtstärkeparameters. In bestimmten beispielsweise besonders kritischen Phasen eines medizinischen Eingriffs wird eine höhere Lichtstärke benötigt als in anderen Phasen. Aus diesem Grund wird insbesondere für jede Phase des medizinischen Eingriffs jeweils der Wert des Lichtstärkeparameters, der Parametersatz und der wenigstens eine externe Eingriffsparameter erfasst.

Der Nutzerparameter umfasst oder kennzeichnet Informationen über ein Nutzungsverhalten und/oder Vorlieben des medizinischen Personals. Auf diese Weise wird beispielsweise berücksichtigt, dass ein erster Arzt während eines bestimmten medizinischen Eingriffs regelmäßig eine höhere Lichtstärke der Lichtquelle einstellt als ein zweiter Arzt.

Der Patientenparameter umfasst oder charakterisiert Informationen, die sich auf den Wert des Lichtstärkeparameters während eines medizinischen Eingriffs am Patienten auswirken können. Dazu gehören beispielsweise ein allgemeiner Gesundheitszustand, ein Körpermassenindex (BMI) und/oder auch ein Körperfettanteil des Patienten.

Der Unterstützungssystemparameter charakterisiert den Einfluss eines klinischen Systems zur Entscheidungsunterstützung. Ein solches System wird vielfach auch als CDS-System (Clinical Decision Support-System) bezeichnet. Dieses System kann indirekt Einfluss auf die Lebensdauer der Lichtleitkomponenten nehmen, da es dem medizinischen Personal Handlungsvorschläge oder Handlungsanweisungen gibt. Das Verhalten des medizinischen Personals führt wiederum direkt zu einer Veränderung der Verschleißsituation.

Gemäß einer weiteren vorteilhaften Ausführungsform ist das medizinische System dadurch fortgebildet, dass die Datenverarbeitungseinheit dazu eingerichtet ist, den externen Eingriffsparameter von einem Krankenhausinformationssystem und/oder von einer von medizinischem Personal bedienbaren Eingabevorrichtung zu empfangen.

Es ist ferner insbesondere vorgesehen, dass die Datenverarbeitungseinheit dazu eingerichtet ist, den Lichtstärkeparameterwert zu empfangen.

Der Lichtstärkeparameterwert, der Lichtleitkomponenten-Parametersatz und der externe Eingriffsparameter werden an die Datenverarbeitungseinheit übermittelt. Der Lichtstärkeparameterwert wird beispielsweise über eine Schnittstelle übermittelt, mit der die Lichtquelle gekoppelt ist und angesteuert wird. Die Parameter werden von einer oder von mehreren Datenquellen übermittelt. Insbesondere werden Identifikationsinformationen des Endoskops und/oder des Kamerakopfes von einem Videoprozessor, der mit dem Endoskop und/oder dem Kamerakopf gekoppelt ist, an die Schnittstelle übermittelt und von der Datenverarbeitungseinheit verarbeitet. Identifikationsinformationen des Lichtleitkabels können beispielsweise über RFID-Technologie (Radio Frequency Identification-Technologie) übermittelt werden. Bei der Schnittstelle handelt es sich also beispielsweise um eine RFID-Schnittstelle. Auch die Nutzerdaten des medizinischen Personals werden beispielsweise mittels RFID-Technologie übermittelt.

Die Eingabevorrichtung ist insbesondere eine tragbare oder in einem Operationssaal fest installierte Vorrichtung, über die das medizinische Personal beispielsweise die Nutzerdaten übermittelt. Die, vorzugsweise drahtlose, Übermittlung geschieht ebenfalls insbesondere mittels RFID-Technologie oder anderer Technologien wie z.B. WLAN (Wireless Local Area Network). Weitere externe Eingriffsparameter werden insbesondere direkt von dem Krankenhausinformationssystem an die Datenverarbeitungseinheit übermittelt. Zu diesem Zweck ist die Datenverarbeitungseinheit drahtgebunden oder drahtlos mit dem Krankenhausinformationssystem verbunden.

Gemäß einer weiteren vorteilhaften Ausführungsform ist das medizinische System dadurch fortgebildet, dass die Datenverarbeitungseinheit dazu eingerichtet ist, wenigstens zwei verschiedene externe Eingriffsparameter zu erfassen.

Beispielsweise erfasst das medizinische System gleichzeitig die Nutzerdaten eines Operateurs und die Art des medizinischen Eingriffs. Diese Daten können beispielsweise aus dem Krankenhausinformationssystem übermittelt werden. Es ist ebenso vorgesehen, dass die Daten aus unterschiedlichen Datenquellen stammen, beispielsweise kann eine erste Information aus dem Krankenhausinformationssystem bezogen werden, während eine zweite Information über eine RFID-Schnittstelle ausgelesen wird. Durch die Erfassung mehrerer externer Eingabeparameter kann der Einfluss der externen Eingriffsparameter auf den Trend des Lichtstärkeparameters sehr gut bereinigt werden.

Gemäß einer weiteren Ausführungsform ist das medizinische System dadurch fortgebildet, dass die Datenverarbeitungseinheit dazu eingerichtet ist, wenigstens zwei Trends des Lichtstärkeparameters zu bestimmen, denen unterschiedliche Lichtleitkomponenten-Parametersätze zugrunde liegen, wobei die zwei unterschiedlichen Lichtleitkomponenten-Parametersätze Konfigurationen der Lichtleitkomponenten beschreiben, bei denen eine erste Lichtleitkomponente übereinstimmt und sich eine zweite und dritte Lichtleitkomponente unterscheiden, wobei die Datenverarbeitungseinheit dazu eingerichtet ist, eine Wartungsbedarfsmeldung für die erste Lichtleitkomponente zu erzeugen, wenn in allen Trends eine einen vorgebbaren Grenzwert überschreitende Änderung des Lichtstärkeparameters feststellbar ist.

Um zu bestimmen, welche der verwendeten Lichtleitkomponenten wartungsbedürftig ist, werden mehrere Trends betrachtet. Konkret werden zeitliche Trends betrachtet, bei denen sich die Konfiguration der Lichtleitkomponenten ändert. Auf diese Weise kann die Lichtleitkomponente, die für eine Änderung des Lichtstärkeparameters verantwortlich ist, identifiziert werden. Tritt beispielsweise eine Veränderung des Lichtstärkeparameters in den Trends von allen medizinischen Eingriffen auf, in denen eine erste Lichtleitkomponente zum Einsatz kam, so ist dies ein starkes Anzeichen dafür, dass diese Komponente wartungsbedürftig ist, weil ein Teil der Lichtleitfasern in der ersten Lichtleitkomponente gebrochen ist.

Die Aufgabe wird zudem gelöst durch ein Verfahren zum Ermitteln eines Wartungsbedarfs einer Lichtleitkomponente eines medizinischen Systems nach Anspruch 5, umfassend eine Lichtquelle, ein Lichtleitkabel, einen Kamerakopf und ein Endoskop als Lichtleitkomponenten, wobei durch die Lichtleitkomponenten jeweils ein Bündel von Lichtleitfasern geführt ist, und wobei das Verfahren durch die folgenden Verfahrensschritte fortgebildet ist:
Erfassen der folgenden Daten während eines medizinischen Eingriffs und abspeichern dieser Daten als historische Daten:
   - einen Lichtstärkeparameterwert der Lichtquelle, der mit einer während des medizinischen Eingriffs von der Lichtquelle abgegebenen Lichtstärke korreliert,
   - einen Lichtleitkomponenten-Parametersatz, der eine Konfiguration der während des medizinischen Eingriffs zum Einsatz kommenden Lichtleitkomponenten beschreibt,
   - einen externen Eingriffsparameter, der Art des medizinischen Eingriffs beschreibt,
wobei das Verfahren ferner die folgenden Schritte umfasst:
   - Berechnen eines Trends des Lichtstärkeparameters aus den historischen Daten von wenigstens zwei medizinischen Eingriffen mit gleichem Lichtleitkomponenten-Parametersatz und gleichen externen Eingriffsparametern,
   - Erzeugen einer Wartungsbedarfsmeldung, wenn in dem Trend eine einen vorgebbaren Grenzwert überschreitende Abweichung festgestellt wird,
   - Ausgeben der Wartungsbedarfsmeldung.

Auf das Verfahren treffen gleiche oder ähnliche Vorteile zu, wie sie bereits zuvor im Hinblick auf das medizinische System erwähnt wurden. Im Rahmen der Erfindung ist es in einer weiteren Ausgestaltung vorgesehen, dass bei der Lichtquelle Lichtkomponenten fest eingebaut sind. Außerdem ist im Rahmen der Erfindung es in einem weiteren Aspekt möglich, dass der Kamerakopf ohne Lichtleitkomponenten ausgebildet ist, wobei dann beispielweise die Lichtleitkomponenten separat am Endoskop, z.B. Laparoskop, angeschlossen werden.

Gemäß einer vorteilhaften Ausführungsform ist das Verfahren dadurch fortgebildet, dass als externer Eingriffsparameter einer oder mehrere der folgenden Parameter erfasst werden: ein Phasenparameter, der eine Phase des medizinischen Eingriffs kennzeichnet, ein Nutzerparameter, der Nutzerdaten des den medizinischen Eingriff durchführenden medizinischen Personals kennzeichnet, ein Patientenparameter, der für einen Patienten charakteristische Daten umfasst, ein Unterstützungssystemparameter, der wenigstens einen Parameter eines Systems zur medizinischen Entscheidungsunterstützung umfasst.

Insbesondere ist ferner vorgesehen, dass der externe Eingriffsparameter von einem Krankenhausinformationssystem und/oder von einer von medizinischem Personal bedienbaren Eingabevorrichtung empfangen wird.

Gemäß einer weiteren Ausführungsform ist vorgesehen, dass wenigstens zwei zeitliche Trends des Lichtstärkeparameters berechnet werden, denen unterschiedliche Lichtleitkomponenten-Parametersätze zugrunde liegen, wobei die zwei unterschiedlichen Lichtleitkomponenten-Parametersätze Konfigurationen von Lichtleitkomponenten beschreiben, bei denen eine erste Lichtleitkomponente übereinstimmt und sich eine zweite und dritte Lichtleitkomponente unterscheiden, wobei eine Wartungsbedarfsmeldung für die erste Lichtleitkomponente erzeugt wird, wenn in allen Trends eine einen vorgebbaren Grenzwert überschreitende Änderung des Lichtstärkeparameters festgestellt wird.

Weitere Merkmale der Erfindung werden aus der Beschreibung erfindungsgemäßer Ausführungsformen zusammen mit den Ansprüchen und den beigefügten Zeichnungen ersichtlich. Erfindungsgemäße Ausführungsformen können einzelne Merkmale oder eine Kombination mehrerer Merkmale erfüllen.

Im Rahmen der Erfindung sind Merkmale, die mit "insbesondere" oder "vorzugsweise" gekennzeichnet sind, als fakultative Merkmale zu verstehen.

Die Erfindung wird nachstehend ohne Beschränkung des allgemeinen Erfindungsgedankens anhand von Ausführungsbeispielen unter Bezugnahme auf die Zeichnungen beschrieben, wobei bezüglich aller im Text nicht näher erläuterten erfindungsgemäßen Einzelheiten ausdrücklich auf die Zeichnungen verwiesen wird. Es zeigen:
- Fig. 1: eine schematisch vereinfachte Darstellung eines Endoskops, eines Kamerakopfes, eines Lichtleitkabels und einer Lichtquelle,
- Fig. 2: eine schematisch vereinfachte Darstellung eines medizinischen Systems zur Ermittlung eines Wartungsbedarfs von Lichtleitkomponenten.

In den Zeichnungen sind jeweils gleiche oder gleichartige Elemente und/oder Teile mit denselben Bezugsziffern versehen, so dass von einer erneuten Vorstellung jeweils abgesehen wird.

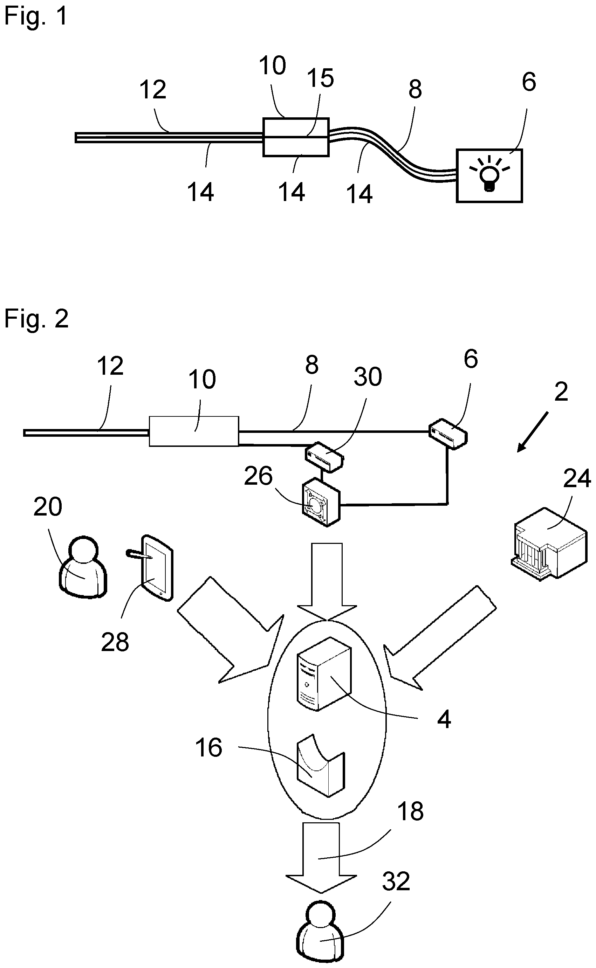

Fig. 1 zeigt in einer schematisch vereinfachten Darstellung ein Endoskop 12, bei dem es sich beispielhaft um ein Videoendoskop mit einem starren Endoskopschaft handelt. Das Endoskop 12 kann ebenso mit einem flexiblen Endoskopschaft ausgeführt sein. Das Endoskop 12 ist an einen schematisch dargestellten Kamerakopf 10 angeschlossen. Der Kamerakopf 10 ist über ein Lichtleitkabel 8 mit einer Lichtquelle 6 verbunden. Durch das Lichtleitkabel 8, den Kamerakopf 10 und das Endoskop 12 sind jeweils Bündel von Lichtleitfasern 15 geführt, mittels derer das von der Lichtquelle 6 erzeugte Licht zu einem distalen Ende des Endoskops 12 geführt wird. Auf diese Weise lässt sich während eines medizinischen Eingriffs ein Blickfeld des Endoskops 12 ausleuchten. Das Lichtleitkabel 8, der Kamerakopf 10 und das Endoskop 12 werden als Lichtleitkomponenten 14 bezeichnet.

Durch mechanischen Bruch einzelner Lichtleitfasern 15 in den Lichtleitkomponenten 14 verringert sich im Laufe der Zeit die Helligkeit, die bei gleichbleibender Lichtstärke der Lichtquelle 6 am distalen Ende des Endoskops 12 emittiert wird. Aus diesem Grund ist es erforderlich, die Lichtleitkomponenten zu warten, um eine optimale Funktionalität sicherzustellen. Jedoch lässt sich der Bruch der Lichtleitfasern 15 und somit der Wartungsbedarf nicht ohne Weiteres feststellen. Eine Abnahme der Helligkeit kann das medizinische Personal in vielen Fällen nicht unmittelbar feststellen. Zum einen unterliegt eine solche Beobachtung einer starken subjektiven Komponente, zum anderen erfolgt die Abnahme der Helligkeit vielfach schleichend über einen längeren Zeitraum.

In Fig. 2 ist ein medizinisches System 2 gezeigt, welches dazu eingerichtet ist, den Wartungsbedarf der Lichtleitkomponenten 14 zuverlässig zu ermitteln. Das medizinische System 2 umfasst eine Datenverarbeitungseinheit 4, beispielsweise einen Computer, die während medizinischer Eingriffe historische Daten sammelt. In Fig. 2 ist dies durch die auf die Datenverarbeitungseinheit 4 gerichteten Pfeile angedeutet. Zu den historischen Daten zählt ein Lichtstärkeparameterwert der Lichtquelle 6, mit dem diese während des medizinischen Eingriffs betrieben wird. Als Lichtstärkeparameterwert wird ein Parameter gewählt, der beispielsweise linear mit der Lichtstärke der Lichtquelle 6 korreliert. Ein geeigneter Lichtstärkeparameterwert ist eine Stromstärke, mit der die Lichtquelle 6 betrieben wird.

Der Lichtstärkeparameterwert wird über eine Schnittstellenvorrichtung 26 an die Datenverarbeitungseinheit 4 übermittelt. Die Schnittstellenvorrichtung 26 ist zu diesem Zweck mit der Lichtquelle 6 verbunden. Obwohl in Fig. 2 als drahtgebundene Verbindung dargestellt, kann diese auch drahtlos ausgestaltet sein. Auch die Datenübertragung von der Schnittstellenvorrichtung zu der Datenverarbeitungseinheit 4 kann drahtgebunden oder drahtlos erfolgen.

Identifikationsinformationen des Endoskops 12 und/oder des Kamerakopfes 10 werden von einem Videoprozessor 30, der mit dem Endoskop 12 und/oder dem Kamerakopf 10 verbunden ist, drahtlos oder drahtgebunden an die Schnittstellenvorrichtung 26 übermittelt, welche die Informationen an die Datenverarbeitungseinheit 4 weiterleitet. Identifikationsinformationen des Lichtleitkabels 8 werden beispielsweise mittels RFID-Technologie oder ebenfalls mittels der Schnittstellenvorrichtung 26 übermittelt. Diese Identifikationsinformationen bilden beispielhaft einen Lichtleitkomponenten-Parametersatz, der die Konfiguration der Lichtleitkomponenten 14 beschreibt.

Zusätzlich zur Erfassung des Lichtstärkeparameterwerts und des Lichtleitkomponenten-Parametersatzes wird ein externer Eingriffsparameter erfasst. Der externe Eingriffsparameter kann wiederum einen oder mehrere Parameter umfassen. Der externe Eingriffsparameter wirkt sich auf den Lichtstärkeparameterwert während eines medizinischen Eingriffs aus, so dass er bei der Auswertung des Lichtstärkeparameterwerts berücksichtigt werden muss.

Parameter, die einzeln oder gemeinsam den externen Eingriffsparameter bilden, sind beispielsweise der Eingriffstyp des medizinischen Eingriffs und/oder die Phase des medizinischen Eingriffs. Weiterhin gehören zu den externen Eingriffsparametern Nutzerdaten des medizinischen Personals 20, das den medizinischen Eingriff durchführt. So bevorzugt ein erster Arzt beispielsweise ein helleres Bild als ein zweiter Arzt. Dies wird in einem Nutzerparameter abgebildet. Auch Patientencharakteristika haben einen Einfluss auf den Wert des Lichtstärkeparameters, beispielsweise der Körpermassenindex (BMI). Dieser Einfluss wird in einem Patientenparameter abgebildet. Wird ein System zur Entscheidungsunterstützung, also ein sogenanntes CDS-System, eingesetzt, so werden ebenfalls Parameter dieses Systems als externe Eingriffsparameter erfasst. Dies ist darauf zurückzuführen, dass sich die Parameter des Systems auf eine Lebensdauer der Lichtleitkomponenten 14 auswirken.

Viele dieser externen Eingriffsparameter, beispielsweise betreffend die Patientencharakteristika oder die Parameter des Systems zur Entscheidungsunterstützung, werden von einem Krankenhausinformationssystem 24 an die Datenverarbeitungseinheit 4 übermittelt. Andere externe Eingriffsparameter, die nicht in dem Krankenhausinformationssystem 24 gespeichert sind, werden von dem medizinischen Personal 20 erfasst und an die Datenverarbeitungseinheit 4 übermittelt. Hierzu kommt beispielsweise eine Eingabevorrichtung 28 zum Einsatz, bei der es sich sowohl um eine tragbare Vorrichtung oder eine fest im Operationssaal installierte Vorrichtung handeln kann. In dem in Fig. 2 gezeigten Beispiel handelt es sich um einen Tabletcomputer. Zu den externen Eingriffsparametern, die vom medizinischen Personal 20 übermittelt werden, zählen beispielsweise die Nutzerdaten des medizinischen Personals 20.

Die Lichtstärkeparameterwerte, die Lichtleitkomponenten-Parametersätze sowie die externen Eingriffsparameter bilden gemeinsam einen Datensatz des medizinischen Eingriffs. Aus den historischen Daten mehrerer medizinischer Eingriffe berechnet die Datenverarbeitungseinheit 4 einen oder mehrere Trends 16 des Lichtstärkeparameterwerts. Die Trends 16 werden um die Einflüsse der externen Eingriffsparameter bereinigt, beispielsweise indem in jedem zeitlichen Verlauf eines Trends 16 ausschließlich historische Daten mit gleichen externen Eingriffsparametern berücksichtigt werden. Um den Wartungsbedarf der Lichtleitkomponenten 14 ermitteln zu können, werden zudem für jeden Trend 16 nur historische Daten berücksichtigt, die mit der gleichen Konfiguration der Lichtleitkomponenten 14 erfasst wurden.

Aus den so ermittelten zeitlichen Trends 16 lässt sich der Wartungsbedarf der Lichtleitkomponenten 14 feststellen. Dazu wird ausgewertet, ob sich der Lichtstärkeparameterwert mit der Zeit ändert, insbesondere ansteigt, und diese Änderung einen vorgebbaren Grenzwert übersteigt. Der Grenzwert wird insbesondere groß genug festgelegt, so dass eine zufällige oder statistische Änderung nicht dazu führt, dass eine Wartungsbedarfsmeldung ausgegeben wird. Eine Änderung des Lichtstärkeparameters, die den Grenzwert übersteigt, weist darauf hin, dass eine gewisse Anzahl von Lichtleitfasern 15 in den Lichtleitkomponenten 14 nicht mehr intakt und beispielsweise gebrochen ist. Um die daraus resultierende verringerte Helligkeit, die am distalen Ende des Endoskops 12 abgegeben wird, auszugleichen, wird das medizinische Personal 20 die Lichtstärke der Lichtquelle 6 erhöhen. Dies führt zu einem Anstieg des Lichtstärkeparameters im Trend 16 - ein Anzeichen für einen Wartungsbedarf der Lichtleitkomponenten 14.

Erkennt die Datenverarbeitungseinheit 4 eine Änderung des Lichtstärkeparameterwerts in den Trends 16, so erzeugt sie eine Wartungsbedarfsmeldung 18. Diese Wartungsbedarfsmeldung 18 wird beispielsweise dem technischen Personal 32 zur Kenntnis gebracht. Bei dem technischen Personal 32 handelt es sich beispielsweise um krankenhauseigenes Personal oder um externes Personal, beispielsweise des Herstellers des medizinischen Systems 2. Die Wartungsbedarfsmeldung 18 ist beispielsweise eine elektronische Nachricht. Alternativ oder zusätzlich kann die Wartungsbedarfsmeldung 18 direkt an den Lichtleitkomponenten 14 ausgegeben werden, beispielsweise durch Aufleuchten einer Signallampe oder dgl. direkt an den Lichtleitkomponenten 14.

Um festzustellen, welche der Lichtleitkomponenten 14 wartungsbedürftig ist, werden mehrere Trends 16 analysiert, deren Lichtstärkeparameterwert mit jeweils unterschiedlichen Konfigurationen von Lichtleitkomponenten 14 erfasst wurden. Beispielsweise werden mehrere Trends 16 analysiert, deren Lichtstärkeparameterwert mit dem gleichen Endoskop 12, aber mit unterschiedlichen Kameraköpfen 10 und Lichtleitkabeln 8 erfasst wurden. Auf diese Weise lässt sich feststellen, ob eine Änderung des Lichtstärkeparameterwerts tatsächlich auf defekte Lichtleitfasern 15 in dem Endoskop 12 zurückzuführen ist, oder ob eine der anderen Lichtleitkomponenten 14 beschädigt ist.

Alle genannten Merkmale, auch die den Zeichnungen allein zu entnehmenden sowie auch einzelne Merkmale, die in Kombination mit anderen Merkmalen offenbart sind, werden allein und in Kombination als erfindungswesentlich angesehen. Erfindungsgemäße Ausführungsformen können durch einzelne Merkmale oder eine Kombination mehrerer Merkmale erfüllt sein.

### Bezugszeichenliste

- 2: medizinisches System
- 4: Datenverarbeitungseinheit
- 6: Lichtquelle
- 8: Lichtleitkabel
- 10: Kamerakopf
- 12: Endoskop
- 14: Lichtleitkomponenten
- 15: Lichtleitfasern
- 16: Trend
- 18: Wartungsbedarfsmeldung
- 20: medizinisches Personal
- 24: Krankenhausinformationssystem
- 26: Schnittstellenvorrichtung
- 28: Eingabevorrichtung
- 30: Videoprozessor
- 32: technisches Personal

## Patentansprüche

1. Medizinisches System (2), umfassend eine Datenverarbeitungseinheit (4) und Lichtleitkomponenten (14), nämlich eine Lichtquelle (6), ein Lichtleitkabel (8), einen Kamerakopf (10) und ein Endoskop (12), durch die jeweils ein Bündel von Lichtleitfasern (15) geführt ist, wobei die Datenverarbeitungseinheit (2) dazu eingerichtet ist, einen Wartungsbedarf der Lichtleitkomponenten (14) zu ermitteln, indem sie während eines medizinischen Eingriffs die folgenden Daten erfasst und als historische Daten abspeichert:
- einen Lichtstärkeparameterwert der Lichtquelle (6), der mit einer während des medizinischen Eingriffs von der Lichtquelle (6) abgegebenen Lichtstärke korreliert,
- einen Lichtleitkomponenten-Parametersatz, der eine Konfiguration der während des medizinischen Eingriffs zum Einsatz kommenden Lichtleitkomponenten (14) beschreibt,
- ein externer Eingriffsparameter, der Art des medizinischen Eingriffs beschreibt,
wobei die Datenverarbeitungseinheit (4) ferner dazu eingerichtet ist, aus den historischen Daten von wenigstens zwei medizinischen Eingriffen mit gleichem Lichtleitkomponenten-Parametersatz und gleichen externen Eingriffsparametern einen Trend (16) des Lichtstärkeparameters zu ermitteln und eine Wartungsbedarfsmeldung (18) zu erzeugen, und wenn in dem Trend (16) eine einen vorgebbaren Grenzwert überschreitende Abweichung feststellbar ist, die Wartungsbedarfsmeldung (18) auszugeben.

2. Medizinisches System (2) nach Anspruch 1, **dadurch gekennzeichnet, dass** der externe Eingriffsparameter einen oder mehrere der folgenden Parameter umfasst:
- einen Phasenparameter, der eine Phase des medizinischen Eingriffs kennzeichnet,
- einen Nutzerparameter, der Nutzerdaten des den medizinischen Eingriff durchführenden medizinischen Personals (20) kennzeichnet,
- einen Patientenparameter, der für einen Patienten charakteristische Daten umfasst,
- einen Unterstützungssystemparameter, der wenigstens einen Parameter eines Systems zur medizinischen Entscheidungsunterstützung umfasst.

3. Medizinisches System (2) nach Anspruch 1 oder 2, **dadurch gekennzeichnet, dass** die Datenverarbeitungseinheit (4) dazu eingerichtet ist, den externen Eingriffsparameter von einem Krankenhausinformationssystem (24) und/oder von einer von medizinischem Personal (20) bedienbaren Eingabevorrichtung (28) zu empfangen.

4. Medizinisches System (2) nach einem der Ansprüche 1 bis 3, **dadurch gekennzeichnet, dass** die Datenverarbeitungseinheit (4) dazu eingerichtet ist, wenigstens zwei Trends (16) des Lichtstärkeparameters zu bestimmen, denen unterschiedliche Lichtleitkomponenten-Parametersätze zugrunde liegen, wobei die zwei unterschiedlichen Lichtleitkomponenten-Parametersätze Konfigurationen der Lichtleitkomponenten (14) beschreiben, bei denen eine erste Lichtleitkomponente (14) übereinstimmt und sich eine zweite und dritte Lichtleitkomponente (14) unterscheiden, wobei die Datenverarbeitungseinheit (4) dazu eingerichtet ist, eine Wartungsbedarfsmeldung (18) für die erste Lichtleitkomponente (14) zu erzeugen, wenn in allen Trends (16) eine einen vorgebbaren Grenzwert überschreitende Änderung des Lichtstärkeparameters feststellbar ist.

5. Verfahren zum Ermitteln eines Wartungsbedarfs einer Lichtleitkomponente (14) eines medizinischen Systems (2), umfassend eine Lichtquelle (6), ein Lichtleitkabel (8), einen Kamerakopf (10) und ein Endoskop (12) als Lichtleitkomponenten (14), wobei durch die Lichtleitkomponenten (14) jeweils ein Bündel von Lichtleitfasern (15) geführt ist, das Verfahren umfassend die folgenden Verfahrensschritte:
Erfassen der folgenden Daten während eines medizinischen Eingriffs und abspeichern dieser Daten als historische Daten:
- einen Lichtstärkeparameterwert der Lichtquelle (6), der mit einer während des medizinischen Eingriffs von der Lichtquelle (6) abgegebenen Lichtstärke korreliert,
- einen Lichtleitkomponenten-Parametersatz, der eine Konfiguration der während des medizinischen Eingriffs zum Einsatz kommenden Lichtleitkomponenten (14) beschreibt,
- einen externen Eingriffsparameter, der Art des medizinischen Eingriffs beschreibt,
wobei das Verfahren ferner die folgenden Schritte umfasst:
- Berechnen eines Trends (16) des Lichtstärkeparameters aus den historischen Daten von wenigstens zwei medizinischen Eingriffen mit gleichem Lichtleitkomponenten-Parametersatz (14) und gleichen externen Eingriffsparametern,
- Erzeugen einer Wartungsbedarfsmeldung (18), wenn in dem Trend (16) eine einen vorgebbaren Grenzwert überschreitende Abweichung festgestellt wird,
- Ausgeben der Wartungsbedarfsmeldung (18).

6. Verfahren nach Anspruch 5, **dadurch gekennzeichnet, dass** als externer Eingriffsparameter einer oder mehrere der folgenden Parameter erfasst werden:
- ein Phasenparameter, der eine Phase des medizinischen Eingriffs kennzeichnet,
- ein Nutzerparameter, der Nutzerdaten des den medizinischen Eingriff durchführenden medizinischen Personals (20) kennzeichnet,
- ein Patientenparameter, der für einen Patienten charakteristische Daten umfasst,
- ein Unterstützungssystemparameter, der wenigstens einen Parameter eines Systems zur medizinischen Entscheidungsunterstützung umfasst.

7. Verfahren nach Anspruch 5 oder 6, **dadurch gekennzeichnet, dass** der externe Eingriffsparameter von einem Krankenhausinformationssystem (24) und/oder von einer von medizinischem Personal (20) bedienbaren Eingabevorrichtung (28) empfangen wird.

8. Verfahren nach einem der Ansprüche 5 bis 7, **dadurch gekennzeichnet, dass** wenigstens zwei zeitliche Trends (16) des Lichtstärkeparameters berechnet werden, denen unterschiedliche Lichtleitkomponenten-Parametersätze zugrunde liegen, wobei die zwei unterschiedlichen Lichtleitkomponenten-Parametersätze Konfigurationen von Lichtleitkomponenten (14) beschreiben, bei denen eine erste Lichtleitkomponente (14) übereinstimmt und sich eine zweite und dritte Lichtleitkomponente (14) unterscheiden, wobei eine Wartungsbedarfsmeldung (18) für die erste Lichtleitkomponente (14) erzeugt wird, wenn in allen Trends (16) eine einen vorgebbaren Grenzwert überschreitende Änderung des Lichtstärkeparameters (14) festgestellt wird.

## Claims

1. A medical system (2), comprising a data processing unit (4) and optical waveguide components (14), namely a light source (6), an optic fiber cable (8), a camera head (10) and an endoscope (12), through each of which a bundle of optical fibers (15) is guided, wherein the data processing unit (2) is configured to determine a maintenance requirement of the optical waveguide components (14) by capturing the following data during a medical procedure and storing said data as historical data:
- a light intensity parameter value of the light source (6), which correlates with a light intensity emitted by the light source (6) during the medical procedure,
- an optical waveguide component parameter set which describes a configuration of the optical waveguide components (14) utilized during the medical procedure,
- an external procedure parameter which describes the type of medical procedure,
wherein the data processing unit (4) is further configured to determine a trend (16) of the light intensity parameter from the historical data of at least two medical procedures having the same optical waveguide component parameter set and the same external procedure parameters and to generate a maintenance requirement message (18) and, if a deviation which exceeds a predefinable limit value can be ascertained in the trend (16), to output the maintenance requirement message (18).

2. The medical system (2) according to Claim 1, **characterized in that** the external procedure parameter comprises one or more of the following parameters:
- a phase parameter which characterizes a phase of the medical procedure,
- a user parameter which characterizes user data of the medical staff (20) performing the medical procedure,
- a patient parameter which comprises data characteristic of a patient,
- a support system parameter which comprises at least one parameter of a system for medical decision support.

3. The medical system (2) according to Claim 1 or 2, **characterized in that** the data processing unit (4) is configured to receive the external procedure parameter from a hospital information system (24) and/or from an input device (28) which can be operated by medical staff (20).

4. The medical system (2) according to any one of Claims 1 to 3, **characterized in that** the data processing unit (4) is configured to establish at least two trends (16) of the light intensity parameter, which are based on different optical waveguide component parameter sets, wherein the two different optical waveguide component parameter sets describe configurations of the optical waveguide components (14) in which a first optical waveguide component (14) matches and a second and third optical waveguide component (14) differ, wherein the data processing unit (4) is configured to generate a maintenance requirement message (18) for the first optical waveguide component (14) if a change in the light intensity parameter, which exceeds a predefinable limit value, can be ascertained in all of the trends (16).

5. A method for determining a maintenance requirement of an optical waveguide component (14) of a medical system (2), comprising a light source (6), an optic fiber cable (8), a camera head (10) and an endoscope (12) as optical waveguide components (14), wherein a bundle of optical fibers (15) is guided in each case through the optical waveguide components (14), the method comprising the following method steps:
capturing the following data during a medical procedure and storing said data as historical data:
- a light intensity parameter value of the light source (6), which correlates with a light intensity emitted by the light source (6) during the medical procedure,
- an optical waveguide component parameter set which describes a configuration of the optical waveguide components (14) utilized during the medical procedure,
- an external procedure parameter which describes the type of medical procedure,
wherein the method further comprises the following steps:
- calculating a trend (16) of the light intensity parameter from the historical data of at least two medical procedures having the same optical waveguide component parameter set (14) and the same external procedure parameters,
- generating a maintenance requirement message (18) if a deviation which exceeds a predefinable limit value is ascertained in the trend (16),
- outputting the maintenance requirement message (18).

6. The method according to Claim 5, **characterized in that** one or more of the following parameters are detected as the external procedure parameter:
- a phase parameter which characterizes a phase of the medical procedure,
- a user parameter which characterizes user data of the medical staff (20) performing the medical procedure,
- a patient parameter which comprises data characteristic of a patient,
- a support system parameter which comprises at least one parameter of a system for medical decision support.

7. The method according to Claim 5 or 6, **characterized in that** the external procedure parameter is received from a hospital information system (24) and/or from an input device (28) which can be operated by medical staff (20).

8. The method according to any one of Claims 5 to 7, **characterized in that** at least two temporal trends (16) of the light intensity parameter are calculated, which are based on different optical waveguide component parameter sets, wherein the two different optical waveguide component parameter sets describe configurations of optical waveguide components (14), in which a first optical waveguide component (14) matches and a second and third optical waveguide component (14) differ, wherein a maintenance requirement message (18) is generated for the first optical waveguide component (14) if a change in the light intensity parameter (14), which exceeds a predefinable limit value, is ascertained in all of the trends (16).

## Revendications

1. Système médical (2) comprenant une unité (4) de traitement de données et des composants (14) conducteurs de lumière, à savoir une source de lumière (6), un câble (8) conducteur de lumière, une tête de caméra (10) et un endoscope (12), à travers lesquels est guidé un faisceau (15) de fibres conductrices de lumière, l'unité (4) de traitement de données étant conçue pour déterminer si les composants (14) conducteurs de lumière nécessitent une maintenance, en enregistrant les données suivantes pendant une intervention médicale et en les sauvegardant sous forme de données historiques :
- une valeur de paramètre d'intensité lumineuse de la source de lumière (6), qui est corrélée à une intensité lumineuse émise par la source de lumière (6) pendant l'intervention médicale,
- un ensemble de paramètres de composants conducteurs de lumière, qui décrit une configuration des composants (14) conducteurs de lumière utilisés pendant l'intervention médicale,
- un paramètre d'intervention externe, qui décrit le type d'intervention médicale,
l'unité (4) de traitement de données est en outre conçue pour déterminer une tendance (16) du paramètre d'intensité lumineuse à partir des données historiques d'au moins deux interventions médicales avec le même ensemble de paramètres de composants conducteurs de lumière et les mêmes paramètres d'intervention externes, et pour générer un message (18) de nécessité de maintenance, et pour, si un écart dépassant une valeur limite prédéfinie peut être constaté dans la tendance (16), émettre le message (18) de nécessité de maintenance.

2. Système médical (2) selon la revendication 1, **caractérisé en ce que** le paramètre d'intervention externe comprend un ou plusieurs des paramètres suivants :
- un paramètre de phase, qui caractérise une phase de l'intervention médicale,
- un paramètre utilisateur, qui caractérise les données utilisateur du personnel médical (20) qui effectue l'intervention médicale,
- un paramètre patient, qui comprend des données caractéristiques d'un patient,
- un paramètre de système d'assistance, qui comprend au moins un paramètre d'un système d'aide à la décision médicale.

3. Système médical (2) selon la revendication 1 ou la revendication 2, **caractérisé en ce que** l'unité (4) de traitement de données est conçue pour recevoir le paramètre d'intervention externe d'un système d'information hospitalier (24) et/ou d'un dispositif de saisie (28) pouvant être utilisé par le personnel médical (20).

4. Système médical (2) selon l'une des revendications 1 à 3, **caractérisé en ce que** l'unité (4) de traitement de données est conçue pour déterminer au moins deux tendances (16) du paramètre d'intensité lumineuse, qui sont basées sur différents ensembles de paramètres de composants conducteurs de lumière, les deux différents ensembles de paramètres de composants conducteurs de lumière décrivant des configurations des composants (14) conducteurs de lumière dans lesquelles un premier composant (14) conducteur de lumière est correspond et un deuxième et un troisième composants (14) conducteurs de lumière sont différents, l'unité (4) de traitement de données étant conçue pour générer un message (18) de nécessité de maintenance pour le premier composant (14) conducteur de lumière lorsqu'une modification du paramètre d'intensité lumineuse dépassant une valeur limite prédéfinie peut être constatée dans toutes les tendances (16).

5. Procédé pour déterminer une nécessité de maintenance d'un composant (14) conducteur de lumière d'un système médical (2), comprenant une source de lumière (6), un câble (8) conducteur de lumière, une tête de caméra (10) et un endoscope (12) en tant que composants (14) conducteurs de lumière, un faisceau (15) de fibres conductrices de lumière étant guidé à travers chacun des composants (14) conducteurs de lumière, le procédé comprenant les étapes suivantes :
enregistrement des données suivantes pendant une intervention médicale et stockage de ces données en tant que données historiques :
- une valeur de paramètre d'intensité lumineuse de la source de lumière (6), qui est corrélée à une intensité lumineuse émise par la source de lumière (6) pendant l'intervention médicale,
- un ensemble de paramètres de composants conducteurs de lumière, qui décrit une configuration des composants (14) conducteurs de lumière utilisés pendant l'intervention médicale,
- un paramètre d'intervention externe, qui décrit le type d'intervention médicale,
le procédé comprenant en outre les étapes suivantes :
- calculer une tendance (16) du paramètre d'intensité lumineuse à partir des données historiques d'au moins deux interventions médicales avec le même ensemble de paramètres de composants (14) conducteurs de lumière et les mêmes paramètres d'intervention externes,
- générer un message (18) de nécessité de maintenance si un écart dépassant une valeur limite prédéfinie est constaté dans la tendance (16),
- émettre le message (18) de nécessité de maintenance.

6. Procédé selon la revendication 5, **caractérisé en ce qu'**un ou plusieurs des paramètres d'intervention externes suivants sont enregistrés :
- un paramètre de phase, qui caractérise une phase de l'intervention médicale,
- un paramètre utilisateur, qui caractérise les données utilisateur du personnel médical (20) qui effectue l'intervention médicale,
- un paramètre de patient, qui comprend des données caractéristiques d'un patient,
- un paramètre de système d'assistance, qui comprend au moins un paramètre d'un système d'aide à la décision médicale.

7. Procédé selon la revendication 5 ou la revendication 6, **caractérisé en ce que** le paramètre d'intervention externe est reçu d'un système d'information hospitalier (24) et/ou d'un dispositif de saisie (28) pouvant être utilisé par le personnel médical (20).

8. Procédé selon l'une des revendications 5 à 7, **caractérisé en ce qu'**au moins deux tendances temporelles (16) du paramètre d'intensité lumineuse sont calculées, sur la base desquelles différents ensembles de paramètres de composants conducteurs de lumière sont définis, les deux ensembles différents de paramètres de composants (14) conducteurs de lumière décrivant des configurations de composants (14) conducteurs de lumière dans lesquelles un premier composant (14) conducteur de lumière est correspond et un deuxième et un troisième composants (14) conducteurs de lumière sont différents, un message (18) de nécessité de maintenance étant généré pour le premier composant (14) conducteur de lumière lorsqu'une modification du paramètre d'intensité lumineuse dépassant une valeur limite prédéfinie est constatée dans toutes les tendances (16).
